(19) Europäisches Patentamt / European Patent Office / Office européen des brevets

(11) **EP 4 398 946 B1**

(12) **EUROPEAN PATENT SPECIFICATION**

(45) Date of publication and mention
of the grant of the patent:
**24.09.2025   Bulletin 2025/39**

(21) Application number: **22776990.8**

(22) Date of filing: **24.08.2022**

(51) International Patent Classification (IPC):
**A61L 24/00** (2006.01)       **A61L 27/42** (2006.01)
**A61L 27/44** (2006.01)       **A61L 27/50** (2006.01)
**A61L 27/54** (2006.01)

(52) Cooperative Patent Classification (CPC):
(C-Sets available)
**A61L 24/0015; A61L 24/001; A61L 24/0063;
A61L 24/0089; A61L 27/425; A61L 27/446;
A61L 27/50; A61L 27/54;** A61L 2300/44;
A61L 2400/06; A61L 2430/02       (Cont.)

(86) International application number:
**PCT/IB2022/057914**

(87) International publication number:
**WO 2023/037192 (16.03.2023 Gazette 2023/11)**

(54) **COMPOSITION OF AT LEAST A RADIOPAQUE AGENT, METHOD FOR ITS PRODUCTION AND FILLING MATERIAL COMPRISING SUCH COMPOSITION**

ZUSAMMENSETZUNG AUS MINDESTENS EINEM RÖNTGENDICHTEN MITTEL, VERFAHREN ZU IHRER HERSTELLUNG UND SOLCH EINE ZUSAMMENSETZUNG UMFASSENDES FÜLLMATERIAL

COMPOSITION D'AU MOINS UN AGENT RADIO-OPAQUE, SON PROCÉDÉ DE PRODUCTION ET MATÉRIAU DE REMPLISSAGE COMPRENANT UNE TELLE COMPOSITION

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:   **08.09.2021   IT 202100023183**

(43) Date of publication of application:
**17.07.2024   Bulletin 2024/29**

(73) Proprietor: **TECRES S.P.A.**
**37066 Sommacampagna (VR) (IT)**

(72) Inventors:
• **FACCIOLI, Giovanni**
**37066 Sommacampagna (Verona) (IT)**
• **SOFFIATTI, Renzo**
**37066 Sommacampagna (Verona) (IT)**

(74) Representative: **Feltrinelli, Secondo Andrea et al**
**APTA S.r.l.**
**Patent Department**
**Via Ca' di Cozzi, 41**
**37124 Verona (IT)**

(56) References cited:
**US-A1- 2003 232 944       US-A1- 2004 157 952
US-A1- 2016 279 289**

• **HOFMANN M P ET AL: "High-strength resorbable brushite bone cement with controlled drug-releasing capabilities", ACTA BIOMATERIALIA, ELSEVIER, AMSTERDAM, NL, vol. 5, no. 1, 1 January 2009 (2009-01-01), pages 43 - 49, XP025804519, ISSN: 1742-7061, [retrieved on 20080826], DOI: 10.1016/J.ACTBIO.2008.08.005**

- **BROUWERS H. J.H. ET AL: "Self-compacting concrete: the role of the particle size distribution", 1 January 2005 (2005-01-01), pages 109 - 118, XP055911642, ISBN: 978-2-912143-62-4, Retrieved from the Internet <URL:https://josbrouwers.bwk.tue.nl/ publications/Conference15.pdf> DOI: 10.1617/ 2912143624.010**
- **ILKKA J ET AL: "Prediction of the compression behaviour of powder mixtures by the Heckel equation", INTERNATIONAL JOURNAL OF PHARMACEUTICS, ELSEVIER, NL, vol. 94, no. 1-3, 21 June 1993 (1993-06-21), pages 181 - 187, XP025565542, ISSN: 0378-5173, [retrieved on 19930621], DOI: 10.1016/0378-5173(93)90022-8**

(52) Cooperative Patent Classification (CPC): (Cont.)

C-Sets
A61L 24/0089, C08L 33/12;
A61L 27/446, C08L 33/12

**Description**

TECHNICAL FIELD OF THE INVENTION

The present invention refers to a composition of at least one radiopaque agent, to a method for its production and to a filling material comprising this composition.

In particular, the present invention relates to a composition in which the at least one radiopaque agent is present both in the form of powder and in the form of granules and in which the filling material is a bone cement to be used in vertebroplasty and/or in orthopaedics.

PRIOR ART

In the medical field, the use of a radiopaque agent to be added to a filling material to be implanted in the human body is known, so as to make the latter visible when the material is subjected to fluoroscopy or radiography, for example X-rays.

However, usually the radiopaque agent is used in the form of a powder, which is added to the filling material in order to make it visible but also so as not to compromise its fluidity and/or mechanical properties.

However, the commonly used filler materials may not be particularly evident in particular conditions, such as when the patient who has to receive the implant, and therefore be subjected to X-rays, is obese.

Patent application US2004157952A1 describes a radiopaque acrylic bone cement for orthopaedic use which comprises a solid phase composed of a mixture of at least one polymer based on polymethyl-acrylate and one or more opaque substances to X-rays, and a liquid phase substantially composed of a mixture of at least one monomer. The radiopaque substance comprises metal particles of tungsten or tantalum coated with a polymeric layer compatible with bone cement, such as an acrylic polymer based on polymethyl methacrylate.

These radiopaque particles have a size between 1 and 100 microns or between 1 and 150 microns, considering the coated version.

Patent application US2016279289 describes a solidifiable acrylic composition comprising a first solid part and a second liquid part, comprising a monomer, suitable for forming a cement which hardens after mixing. The first solid part comprises a first sub-population of acrylic polymer particles and a second sub-population of acrylic polymer particles and a radiopaque filler, which is encapsulated in and/or adsorbed on the particles of the first sub-population and of the second sub-population, even if the latter have a smaller average size than those of the first sub-population.

There is therefore a need to have a radiopaque agent capable of being used in any condition, and capable of making the filling material to which it is added visible with any radiography technique and with any type of patient.

OBJECTS OF THE INVENTION

The present invention has the technical task of improving the state of the art in the field of radiopaque agents and/or filler materials comprising a radiopaque agent.

Within the scope of this technical task, an object of the present invention is to provide a composition of a radiopaque agent which is capable of being visible in any type of fluoroscopic, radiographic situation and/or with any type of patient.

A further object of the present invention is to provide a composition of a radiopaque agent which substantially does not compromise the fluidity and/or the mechanical characteristics of the filler material to which it is added in use.

According to an aspect of the present invention, a radiopaque agent composition according to claim 1 is provided.

A further object of the present invention is to provide a filling material comprising a composition of a radiopaque agent, the radiopaque agent being the radiopaque system according to claim 1, which can be implanted and/or injected and which is visible in any fluoroscopic, radiographic situation and/or any type of patient.

A still further object of the present invention is to provide a filling material comprising a composition of a radiopaque agent, the radiopaque agent being the radiopaque system according to claim 1, capable of maintaining its fluidity properties and/or its mechanical characteristics substantially unaltered, despite the presence of this composition.

According to an aspect of the present invention, a filling material is provided comprising a composition of a radiopaque system and a relative kit according to claims 8 and 13.

A further object of the present invention is to provide a method for obtaining a composition of a radiopaque agent which is simple and fast.

According to an aspect of the present invention, a method according to claim 16 is provided.

The dependent claims refer to preferred and advantageous forms of the invention.

BRIEF DESCRIPTION OF THE DRAWINGS

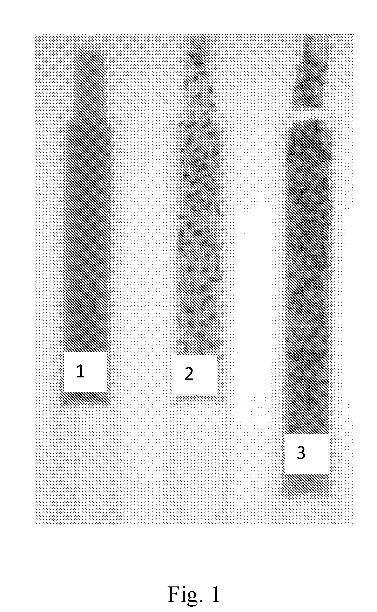

Figure 1 is a radiographic front view of three syringes in which: syringe 1 contains traditional cement, e.g. for vertebroplasty, with fine barium powder (100% barium is fine), syringe 2 contains cement with granular barium (100% of the barium is in granules) while the syringe 3, which is the most evident, contains a cement comprising a radiopaque agent composition according to the present invention (in which the composition comprises for example 33% barium sulphate fine and the remaining 66-67% granular barium sulphate),

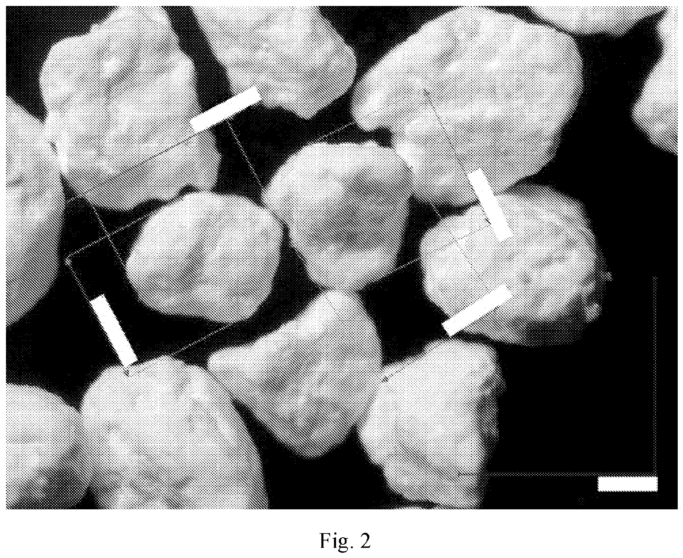

Figure 2 is a photomicrograph illustrating granules of the second radiopaque agent of the composition according to the present invention,

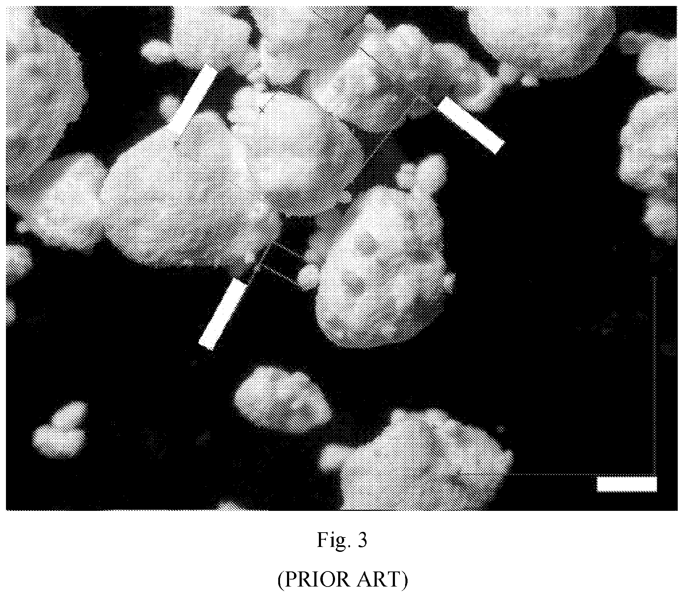

Figure 3 represents a photomicrograph illustrating granules of a prior art radiopaque agent,

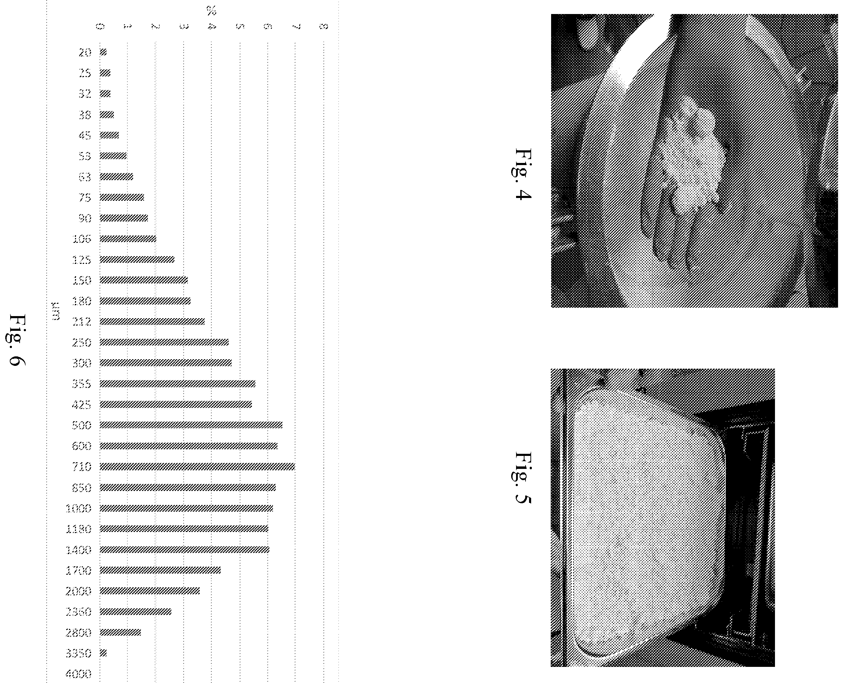

figure 4 represents the powder of a (first or second) radiopaque substance, in particular barium sulphate,

Figure 5 represents the composition according to the present invention extracted from the reactor, i.e. in an intermediate stage of its preparation,

figure 6 represents a graph (graph 1) in which in the abscissa there is the granulometric dimension in microns while in the ordinate there is the quantity in percentage terms, in particular referring to the barium sulphate powder after the crushing step in the hammer mill with 2 mm screen and 1500 RPM speed,

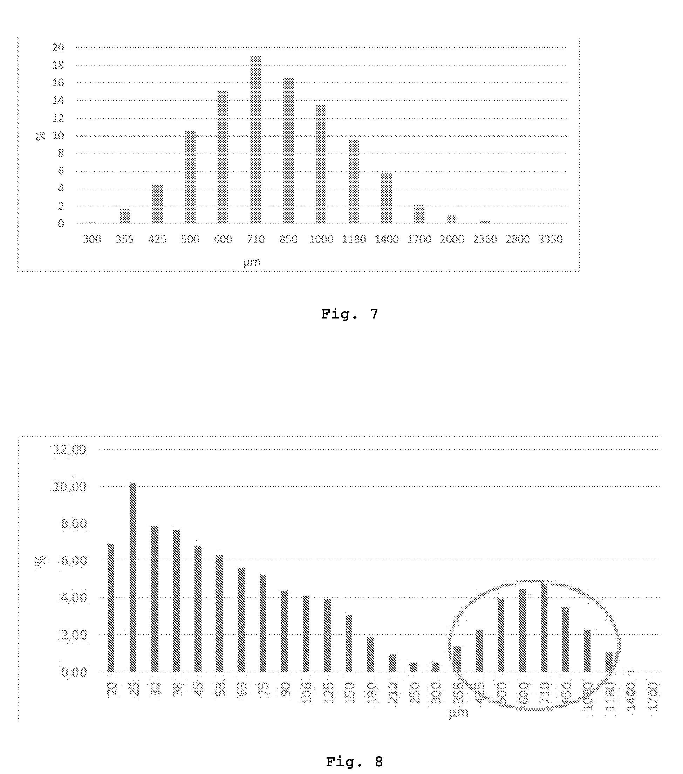

Figure 7 represents a graph (graph 2) in which the granulometric size in microns is present in the abscissa while the quantity in percentage terms is present in the ordinate, in particular referring to the barium sulphate powder after the sieving step on a pair of sieves of 600 microns and 850 microns,

figure 8 represents a graph (graph 3) in which in the abscissa there is the granulometric dimension in microns while in the ordinate there is the quantity in percentage terms, in particular of a bone cement comprising the composition according to the present invention (in detail a cement bone comprising PMMA powder and barium sulphate granules according to the present invention, the latter enclosed in the circle shown, as well as barium sulphate in the form of a fine powder),

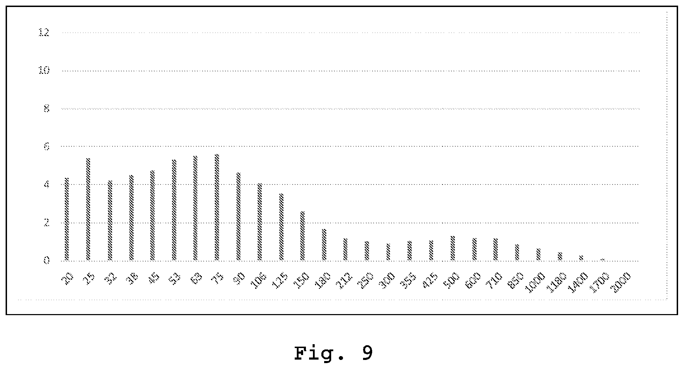

figure 9 represents a graph (graph 4) in which in the abscissa there is the granulometric dimension in microns while in the ordinate there is the quantity in percentage terms, in particular of a bone cement comprising a radiopaque agent in fine powder (in detail a cement bone comprising only PMMA powder and 30% fine powdered barium sulphate powder).

EMBODIMENTS OF THE INVENTION

The radiopaque system serves to make visible, for example, when added to it, a filling material that is injected and/or implanted in a patient, for example by means of a vertebroplasty and/or orthopaedic technique, when the patient is subjected to x-ray or radiation, such as X-rays.

X-rays or gamma rays, for example, find it more difficult to overcome or transit through materials of high density such as metals or their salts or, for the same material, when this has a greater thickness than the same material of reduced thickness. Therefore, by increasing the thickness of the radiopaque agent, a better opacity of the agent itself is obtained, as the fraction of energy of the rays absorbed per centimetre of material passed through increases (also depending on the type of material, its thickness and the wavelength of incident radiation).

The composition of radiopaque agent according to the present invention is therefore a radiopaque system, able in use to be added to a filling material for the human body and able to make this filling material radiopaque when subjected to radiography or radiation, for example X-rays.

Therefore, in the present discussion, when a radiopaque agent composition is mentioned, reference is made to this radiopaque system, and not also to the material used to form the filler material for the human body.

In at least one version of the present invention, the composition according to the present invention consists of at least a first radiopaque agent in the form of a powder and at least a second radiopaque agent in the form of granules, as defined in claim 1.

The first radiopaque agent comprises at least a first radiopaque substance and the second radiopaque agent comprises at least a second radiopaque substance in the form of particles.

The first radiopaque substance is the same as the second radiopaque substance or is different from the latter.

The granules of the second radiopaque agent have an overall larger size than the powder of the first radiopaque agent, as will be better clarified hereinafter.

In particular, the granules of the second radiopaque agent have at least a size or a diameter between 600 microns and 850 microns and comprise (or are formed by) the particles of said at least one second radiopaque substance and - as will be better indicated below - an adhesive substance, able to hold together and/or glue and/or compact and/or aggregate the particles of this at least one second radiopaque substance.

The granules of the second radiopaque agent are not drugs (which must be soluble in the human body) but perform a different function, i.e. they are radiographic markers and must perform this function indefinitely over time. Therefore, they do not have to be soluble.

Based on this, the at least one second radiopaque substance and the adhesive substance are insoluble in water and/or in aqueous solutions and/or in organic solvents and/or in the human body.

The at least one first radiopaque agent in the form of a powder, also called a fine radiopaque agent, and/or the at least one first radiopaque substance has particles having a substantially regular and/or irregular and/or spherical shape and/or a shape similar to a parallelepiped for example with rounded corners, and/or elongated or oblong, and/or oval or ovoid, and/or cylindrical with rounded bases, and/or a shape similar to a "baguette", and/or needle-like, etc.

In particular, according to at least one version of the present invention, the powder of the at least one first radiopaque agent is formed by random agglomerates of needle-like crystals of the at least one first radiopaque substance (starting or initial). For example, Barium Sulfate fine powder is formed from random agglomerates of Barium Sulphate needle-like crystals about 1 micron or about 0.7 micron in size. Therefore, the fine powder of the at least one first radiopaque agent, at least in this version, has no regular size. According to at least one version of the present invention, the first radiopaque substance which forms the first radiopaque agent is obtained by precipitation of barium sulphate and the crystals are dimensionally very constant. Therefore, the first radiopaque agent comprises or consists of such precipitated Barium Sulphate, which is in the form of a powder.

In that case, the powder of the first radiopaque agent has a size or diameter of between 0.1 micron and 10 micron or between 0.1 and 1.5 micron or between 0.1 micron and 1 micron.

The particles of the at least one first radiopaque agent have a size and/or diameter generally less than 100 microns or less than 250 microns or between 100 and 250 microns. The fact that the powder of the first radiopaque agent is so fine, when added to the filling material to be implanted in the human body, gives it - when subjected to radiation - a homogeneous opacity which is highlighted with a constant gray tone on the radiograph (see for example figure 1). The second radiopaque agent, with its hard granules and of selected dimensions, on the radiograph appear as white specks on a gray background (opaque colour given by the powder of the first radiopaque agent), clearly visible and above all in motion during continuous fluoroscopy. The fact that it is possible to perceive the flow of the filling material (or bone cement) thanks to the movement of the granules of the radiopaque system that has been added to the material itself, constitutes a further safety for the surgeon who can control, for example, the injection of the filling material at the vertebral level, avoiding the risk of likage. As can be easily understood, this injection safety is obtained only thanks to the granules of the second radiopaque agent (for example of barium sulphate) which - being visible on the gray background given by the powder and mobile based to the fluidity of the injected filler material - allow to capture in real time the displacement of the filling material and its correct positioning in the implant site.

The at least one second radiopaque agent, in the form of granules, on the other hand, has granules having a substantially regular and/or irregular and/or spherical shape and/or a shape similar to a parallelepiped, for example with rounded corners, and/or elongated or oblong, and/or oval or ovoid, and/or cylindrical with rounded bases, and/or a shape similar to a "baguette", and/or needle-like, etc.

According to a version of the invention, the at least one second radiopaque agent has granules whose size is based on a Gaussian or Gaussian-like distribution.

According to a version of the invention, the second radiopaque agent is present in a ratio of about 3:1 with respect to the first radiopaque agent, on the total of the radiopaque system according to the present invention.

According to a further version, the second radiopaque agent is present in a ratio of about 2:1 with respect to the first radiopaque agent, on the total of the radiopaque system according to the present invention.

According to a still further version, the second radiopaque agent is present in a ratio of about 4:1 with respect to the first radiopaque agent, on the total of the radiopaque system according to the present invention.

Lastly, the second radiopaque agent can be present in a ratio ranging from about 4:1 to about 1:1 with respect to the first radiopaque agent, on the total of the radiopaque system according to the present invention.

According to an example, the radiopaque composition of the present invention comprises 7 grams of at least a first radiopaque agent and 22 grams of at least a second radiopaque agent, for a total of about 29-30 grams of radiopaque system. **In** this way, excellent visibility properties are obtained.

Similar quantities can also be calculated, on a total of about 30 grams of composition, according to the ratios indicated above. That is to say, out of a total of about 30 grams of composition, the granules of the second radiopaque agent can weigh between 15 grams and 7 grams or between 15 grams and 22 grams or between 7 grams and 22 grams, while the remainder in grams is provided by the powder of the first radiopaque agent. However, for particular application requirements, it is possible to make mixtures different from the point of view of the weight of the powder of the first radiopaque agent and of the granules of the second radiopaque agent (for example Barium) with respect to each example just described.

According to a version of the invention, the granules of the second radiopaque agent are made according to the following method.

At least a second radiopaque substance is provided in the form of particles and a liquid solution of an adhesive substance.

These particles are supplied in the form of a powder.

The at least one second radiopaque substance is supplied in a percentage equal to 70-97% w/w (weight/weight) of the total of the second radiopaque agent while the liquid solution is supplied in a percentage equal to 3-30% w/w of the total of the second radiopaque agent, considering the total of the second radiopaque agent given by the sum by weight of the at least one radiopaque substance and the liquid solution.

As mentioned, the particles in the form of powder of the at least one second radiopaque substance are, for example, needle-like, for example in the form of crystals, for example with a size of about 1 micron.

The liquid solution is used to moisten the powder so that a wet aggregate mass is formed, after combining the liquid solution and the second radiopaque substance.

For example, the powder of the at least one second radiopaque substance is inserted (for example visible in Figure 4) and the liquid solution in a container or reactor where they are mixed, for example by mixing these two components manually or automatically. The liquid solution is prepared by combining at least one solvent (such as for example a polar or non-polar liquid and/or solvent or a mixture between the two types, or water, ethanol, methanol, acetone, ether, hexane, etc.) and the at least one adhesive substance in solid form.

The adhesive substance in solid form comprises a solid, soluble in at least one solvent, such as for example cellulose, polyvinyl alcohol, polyvinylpyrrolidone, carboxymethylcellulose, at least one sugar, polyvinyl acetate and analogues, synthetic and natural gums and resins, etc.

According to a further version of the present invention, the adhesive substance can comprise at least one of an acrylic resin, a methacrylic resin, a styrenic resin or a mixture of at least two of the same.

Such resins can be present in the form of polymer and/or co-polymer and/or a mixture thereof.

In this case, the solvent used can be at least one acrylic, methacrylic or styrenic monomer. The fact that the adhesive substance can be a polymer or a mixture of polymers has the purpose of modulating and/or varying the overall molecular weight of the adhesive substance itself, to regulate the final viscosity of the radiopaque system and/or of the second radiopaque agent and/or the liquid solution of adhesive substance, according to the surgical or patient needs and/or according to the type of granules to be obtained. For example, if a liquid solution with a viscous consistency is desired, it will comprise single or mixed polymers with high molecular weight, if instead a liquid solution with a fluid and/or non-viscous consistency is desired, it will contain a polymer or mixture with low molecular weight.

In this way, it is also possible, for example, to vary the size and/or conformation of the resulting lumps.

The adhesive substance acts as an aggregator for the granules of the at least one second radiopaque agent. It is capable of holding together and/or gluing and/or compacting and/or aggregating the particles in the form of powder of the at least one second radiopaque substance, precisely forming - after further processing - the granules of the second radiopaque agent.

The adhesive substance, in at least one version of the present invention, does not form an external coating for such granules.

Rather, it is homogeneously distributed within these granules.

Thanks to the union with this liquid solution and with this adhesive substance, the powder particles of the at least one second radiopaque substance are aggregated and held together in the form of lumps of variable size, for example having a size from 3 mm up to about 50 mm in diameter: this is the before-mentioned wet aggregate mass (visible, for example, in figure 5).

The powder of the at least one second radiopaque substance is then introduced into the reactor, which is equipped with a stirrer, and the liquid solution is added, for example in regular aliquots. The agitator promotes the rolling of the powder until the lumps of the wet aggregate mass are obtained, which are for example spheroidal. This happens, for example, thanks to the so-called "snow avalanche effect".

The liquid solution contains 70-99% w/w of liquid solvent and 30-1% w/w of adhesive in solid form.

Subsequently the wet aggregate mass is extracted from the reactor and an evaporation step, for example in vacuum, of the solvent takes place in order to dry the lumps of the wet aggregate mass. These lumps solidify and/or consolidate thanks to the presence of the adhesive substance which cannot evaporate. In this way solidified lumps are obtained, which are sent for subsequent processing.

These subsequent processing include a crushing step and a selection step.

The crushing step takes place for example in a mechanical mill, for example with hammers.

The selection step takes place by sieving on a battery of sieves with suitable mesh: the pertinent particle size fractions according to the present invention are selected.

In the embodiments according to claim 16, stacked sieves are 600 microns and 850 microns are used.

In this way, it is possible to select only the granules having at least one size or a diameter less than or equal to 850 microns and at the same time having at least one or two dimensions or a diameter greater than or equal to 600 microns.

The at least one second radiopaque agent is thus obtained in the form of granules, in which the granules have a larger size than the powder and comprise - within them - the adhesive substance.

The liquid solvent evaporates and therefore no trace of it remains in the granules. What remains is instead the adhesive substance in solid form in itself and/or after reaction with the solvent, for example the at least one polymer, which being solid does not evaporate. The percentage of adhesive substance present in the granules is therefore directly proportional to the quantity present in the liquid solution of adhesive substance, also with respect to the quantity of the second radiopaque substance present.

For example, if 900g of second adhesive substance (for example barium sulphate) and 500g of liquid solution formed by 400g of solvent and 100g of adhesive substance in solid form are combined, at the end after evaporating the solvent, you will have a mass of 1000 grams of lumps and/or coarse granules. Such lumps will contain 90% barium and 10% adhesive substance (for example the at least one polymer). The same composition will also have the selected granules at the end.

Therefore, according to the present invention, the second radiopaque agent in the form of granules comprises a percentage of between 1% and 30% by weight of said adhesive substance on the total weight of said second radiopaque agent. For example, the the adhesive substance present in the granules can correspond to approximately 10% by weight of the total weight of the granules and/or of each granule.

Thereafter, the at least one first radiopaque agent is provided and the same is added to the second radiopaque agent.

In at least one version of the present invention, the first radiopaque agent also comprises the adhesive substance. It is obtained for example as indicated below.

**Example of the particle size variations of the at least one radiopaque agent during the various steps of the process.**

Graph 1 shown in Fig. 6 shows the extensive granulometry of the solidified and crushed lumps obtained by crushing, for example by means of a hammer mill. The solidified and crushed lumps have a particle size between 20 microns and approximately 3350 microns. The maximum percentage of these lumps is around a lump size equal to 710 microns, the value before which the curve of graph 1 is increasing and the value beyond which the curve decreases. However, most of these clumps have a particle size greater than 250-300 microns.

Then there is a selection step by sieving, for example on a pair of 600 micron and 850 microns sieves, the solidified and crushed lumps.

In theory, sieving should isolate a granular product with dimensions strictly between 600 and 850 microns. However, as shown in graph 2 (Fig. 7), the product that comes out of this sieving step still has particle sizes lower than 600 microns and higher than 850 microns distributed around the average value (about 710 microns), for example according to a Gaussian or Gaussian-like trend.

Only 30-40% or up to about 50% of the solidified and crushed lumps, after sieving, actually have granules having a size between 600-850 microns. This is because grains that do not have a spherical conformation can pass through the sieves for the larger side or the smaller side, thus causing the particle size distribution shown in Graph 2.

According to at least one version of the invention, the size of the granules which can be found in graph 2 can be between 300 microns and 2800 microns.

The measurements can be made using an analytical instrument called a Laser Granulometer.

The following table (Table 1) analyses the percentages of solidified, crushed and sieved lumps of 6 different lots of composition selected with the two production sieves stacked 600 +850 $\mu$m. The solidified, crushed and sieved lumps may also be referred to as granules of the second radiopaque agent in the present discussion.

Each single lot is analysed with analytical sieves with the same nominal meshes of 600 and 850 $\mu$m. Three selected fractions are obtained, namely: less than 600, between 600 and 850, greater than 850 $\mu$m.

Table 1

| Lots of lumps sieved on 600-850 $\mu$m sieves | <600 microns | 600-850 microns | > 850 microns |
|---|---|---|---|
| CU867_media | 34.72% | 33.99% | 31.28% |
| CU874_media | 16.97% | 42.97% | 40.06% |
| CU875_media | 18.40% | 42.23% | 39.37% |
| CU939A_media | 18.64% | 34.12% | 47.23% |
| CU939B_media | 21.87% | 31.45% | 46.611% |
| CU940_media | 20.71% | 30.78% | 48.48% |

As can be seen from the table above, about 20-22% on average of solidified, crushed and sieved lumps have a particle size of less than 600 microns, about 35-36% on average of solidified, crushed and sieved lumps have a particle size between 600 microns and 850 microns while on average 40-42% of solidified, crushed and sieved lumps have a particle size greater than 850 microns.

Therefore, the granules of the second radiopaque agent could have a size and/or a diameter generally comprised between 600 microns and 850 microns, less than 600 microns and/or greater than 850 microns.

However, according to the present invention, the granules of the second radiopaque agent have a size comprised between 600 and 850 microns.

Indeed, during the production of the granules, in the sieving step, only the 600-850 micron fraction is recovered and used in the composition according to the present invention. The two external fractions, i.e. lower than 600 microns and higher than 850 microns, can be discarded and not used in the composition according to the present invention.

As regards the at least one first radiopaque agent, it can be obtained according to the following method.

At least one powdered radiopaque substance is provided, for example starting or initial, which has, for example, needle-like particles, for example in crystals, for example with dimensions of about 1 micron or less than 1 micron.

Such at least one first initial radiopaque substance can be the same or different from the at least one second radiopaque substance which is supplied for obtaining the at least one second radiopaque agent.

This first initial radiopaque substance is sieved so as to recover the fraction having the desired size and/or so as to select particles of this powder of the at least one radiopaque substance. Before or after the sieving step, a step can take place of thickening the powder of the at least one first initial radiopaque substance and/or the particles thereof, for example to form aggregates or agglomerates of larger dimensions than those of the at least one starting radiopaque substance, and thus obtaining the desired size of the fine powder of the at least one first radiopaque agent.

The aggregates or agglomerates may constitute the powder of the at least one first radiopaque agent.

According to at least one version of the invention, this step can be obtained by joining the at least one initial radiopaque substance with a liquid solution containing a solvent and an adhesive substance equal to or different from that described above. Therefore, also the powder of the first radiopaque agent can comprise the particles of the at least one first radiopaque substance held together and/or glued and/or compacted by the adhesive substance, possibly after solidification and/or consolidation and/or evaporation of the solvent present in said liquid solution.

Therefore, this method optionally comprises solidifying and/or consolidating the particles of the at least one first radiopaque substance and/or evaporating any solvents present in the liquid solution, so as to obtain said powder of said at least one first radiopaque agent. The powder of the at least one radiopaque agent, being contacted and comprising the adhesive substance, has a larger size than those of the powder particles of the at least one initial or starting radiopaque substance.

However, in a preferred version of the invention, the first radiopaque substance and/or the first radiopaque agent is devoid of adhesive substance.

In particular, according to at least one version of the present invention, the first radiopaque agent comprises powder obtained from pure barium sulphate, i.e. not treated in any way. In other words, the first radiopaque agent will contain 100% of the first radiopaque substance, for example 100% barium sulphate.

In this version, only the granules of the second radiopaque agent comprise a percentage of adhesive substance. The percentage, as we have seen, can be variable according to needs.

If, in one version of the invention, it is desired to use the fraction of the granules having a size or a diameter of less than 600 microns, in particular of the dimensions indicated above, as the first powdered radiopaque agent, the latter will contain the same percentage of adhesive substance present in the second radiopaque agent in the form of granules (with particle size between 600 and 850 microns), both of which derive from the same production method.

Basically, according to at least one version of the invention, the at least one first radiopaque agent (in fine powder) and the at least one second radiopaque agent (in granules) are two independent materials, either by the nature of the material, or by the their particle size, either by the method of obtaining, or by a combination of the above characteristics.

Therefore, to the second radiopaque agent obtained according to the method indicated above, a first radiopaque agent, of a material equal to or different from the second radiopaque agent, can be added, so as to form the radiopaque system according to the present invention.

According to at least one version of the present invention, therefore, the radiopaque system is formed as follows:

first radiopaque agent in powder form (the powder has a size between 100 and 250 microns or less than 100 microns and/or less than 250 microns): it is formed by particles in the form of powder of at least one first radiopaque substance or by particles in the form of powder of at least one first radiopaque substance held together and/or glued and/or compacted thanks to the adhesive substance, and

second radiopaque agent in granules (the granules have a size between 600 microns and 850 microns): it is formed by particles in the form of powder of at least a second radiopaque substance held together and/or glued and/or compacted thanks to the adhesive substance.

The radiopaque system thus formed, according to the weight ratios indicated between the first radiopaque agent and the second radiopaque agent, is in use to be added to a filling material to be implanted in the human body, such as a bone cement, to make the latter visible to radiation. when subjected to radiographic techniques.

According to a still further example, if necessary, granules having a size between 600 microns and 850 microns of a material different from that of the first and/or second radiopaque agent also present in the composition can be added.

This depends on the type of use to be made of the composition according to the present invention, and on the effect to be obtained.

The filling material, for example a bone filling material, for a damaged bone tissue and/or to fill a bone gap or cavity and/or for the realization or fixing of temporary or permanent prosthetic devices, to which the composition can be added according to the present invention, it is a bone cement to be used in vertebroplasty and/or in orthopaedics. The filling material therefore comprises a plastic polymer and/or an acrylic resin, and/or a polymer based on PMMA and/or styrene and/or acrylic copolymers, and/or a ceramic bone cement, for example based on tricalcium phosphate (TCP) and/or hydroxyapatite, etc. or a mixture thereof.

The radiopaque system according to the present invention is able, as mentioned, to be added in use to the filling material (bone) after realization of the same.

The radiopaque agent composition is therefore a radiopaque agent additive composition for the filler material.

As is known, a filling material can be used in vertebroplasty, so as to be injected into diseased or damaged vertebrae of a patient, or in orthopaedics, to fill bone gaps and/or to fix prostheses and other devices to the patient's bones.

The filler material can be injected into the human body or applied in another way. It solidifies after a certain period of time, depending on its specific composition, so as to become one with the tissue, for example bone, with which it comes into contact.

In these cases, the filling material must be radiopaque in order to be visualized, during its insertion into the human body or even after implantation, and therefore comprises the radiopaque agent composition according to the present invention.

This need is particularly felt in general but even more so when it comes to intervening in obese patients, in which it is very difficult to distinguish the bone cement that is implanted. Therefore, the present invention has succeeded in obtaining a composition of a radiopaque agent and the filling material comprising it which are capable of being more evident in radiography than materials of known use.

As can be seen from Figure 1, in fact, the syringe 1 contains a filling material of the traditional type, in which the filling material is bone cement and comprises only a radiopaque agent of the powder or fine type. Syringe 2, on the other hand, comprises a filling material, for example a bone cement, which contains only a radiopaque agent in the form of granules: this syringe has better radiographic visibility than syringe 1. Finally, syringe 3, which is the most evident, contains a filling material, for example a bone cement, to which the radiopaque agent composition according to the present invention has been added (in which the composition comprises for example 1/3 of a first radiopaque agent, for example fine barium sulphate, with the addition of 2/3 of a second radiopaque agent, e.g. granular barium sulphate). It is therefore evident that the present invention achieves a much better result than the other two, surprisingly with respect to expectations, in which the visibility under X-rays is much improved.

Moreover, thanks to the properties of the composition according to the present invention, the mechanical properties of the filling material are not minimally compromised with respect to the same material comprising a powdered radiopaque agent or not comprising any radiopaque agent.

The radiopaque agent according to the present invention comprises at least one of the following compounds: barium sulphate, zirconium oxide, bismuth salts, tungsten powders, tantalum, etc.

The at least one first radiopaque substance and/or the at least one second radiopaque substance is barium sulphate, zirconium oxide, bismuth salt, tungsten powder, tantalum, etc. or combinations thereof.

In one version of the invention, the radiopaque agent composition is present substantially for 30% by weight of the total weight of the filler material.

In a further version, the radiopaque agent composition is substantially present for 10% by weight of the total weight of the filling material, according to another version it is between 5% and 50% or between 10% and 40% or between 20% and 30% by weight of the total weight of the filling material.

Considering the at least one first radiopaque agent, it can constitute 5-10% by weight or 7% by weight of the total filling material. The at least one second radiopaque agent, on the other hand, can constitute 10-30% by weight or 22% by weight of the total weight of the filling material.

Graph 3 (Fig. 8) shows the granulometry of a bone cement added with the composition according to the present invention: in the round the fraction of the composition according to the present invention is evident, having at least a dimension between 300 microns and 1180 microns and/or granules having at least a size comprised between 600 microns and 850 microns.

The difference is very evident when comparing graph 4 (Fig. 9) in which at least one radiopaque agent only in fine powder is added to the bone cement. It can be seen that the increase of the portion having a size between 300 microns and 1180 microns and/or of the granules having at least a size between 600 microns and 850 microns is completely missing.

If necessary, the filler material may comprise at least one pharmaceutical or medical substance, in addition to the radiopaque agent composition according to the present invention. This substance, if the filling material has the purpose of treating an infection in progress, can be an antibiotic, such as for example gentamicin sulphate or vancomycin, etc., or mixtures thereof.

According to a further version, the substance can be a substance capable of promoting and/or stimulating bone or tissue growth, or a chemotherapeutic agent, etc.

Usually, the system according to the present invention is in dry and/or solid form. This is because both the at least one first radiopaque agent in the form of powder and the at least one second radiopaque agent in the form of granules are in dry and/or solid form.

Thanks to the present invention, to obtain the filling material (bone), the surgeon will only have to add the radiopaque system according to the present invention, wanting already comprising the first radiopaque agent in the form of powder and the second radiopaque agent in the form of granules, to the material filler and/or its part or component in powder form, to obtain the desired material and with much improved radiopaque properties compared to the prior art.

The radiopaque system in dry and/or solid form, therefore, can be added to the powder component of the filling material. In an alternative version, the system in dry and/or solid form is added to the filling material after that for the latter its (possible) phases in powder and liquid have already been combined (the latter being, for example, a liquid monomer and/or a curing agent and/or a gluing agent, etc.).

The at least one second radiopaque agent according to the present invention comprises hard granules. These granules are granules that do not flake when subjected to manual pressure and/or during the mixing steps with the other components of the filling material. In this case, the term "hard", therefore, means not flaky or breakable during the handling and processing steps of these granules.

This feature allows the granules to maintain their shape and size even once implanted in the human body (as shown, for example, in figure 2).

On the other hand, some prior art radiopaque agent granules (shown in Figure 3, for example in the absence of an adhesive substance comparable to that of the present invention), not being as hard as the granules according to the present invention, are flaked and reduced to particles smaller than expected, after their mixing step with the other components of the filling material, or even simply after their handling and/or transport. In this case it is understood that the percentage and/or the size of the granulometry selected by the manufacturer is not maintained during the use of these prior art granules. In this case, even the radiographic visibility characteristics do not correspond to those selected, as some percentages of granules flake off and increase the percentage of powdered radiopaque agent. This implies that with the vibrations due to transport, the granules of prior art (which are clearly visible in radiography) can be reduced in number or completely disappear and nullify their presence in terms of visibility.

On the contrary, the granules of the present invention (comprising particles of at least a second radiopaque substance and an adhesive substance) maintain their dimensions even during use, and therefore the percentage and/or size of granules will be that established a priori, with consequent maintenance also of the visibility implemented during fluoroscopic and/or radiographic techniques.

According to the present invention, the granules have a density between 1.15 and 1.35 $g/cm^3$, or a density between 1.25 and 1.30 $g/cm^3$, or equal to about 1.25 $g/cm^3$, or equal to 1.27-1.28 $g/cm^3$, or equal to 1.277 $g/cm^3$. The flaky granules of the prior art, on the other hand, have a density lower than 1.10 $g/cm^3$, or lower than 1.07 $g/cm^3$.

This density is for example measured by hydrostatic weighing.

According to the present invention, the granules have an average compressive strength of not less than 1000 N corresponding to 10% of compression and/or an average compressive strength of not less than 3000 N corresponding to 20% of compression. These values were determined according to the method indicated below.

The granules of the present invention, for example, have a compressive strength which is approximately 50 times greater than that of the prior art granules.

Therefore, when in the present description it is said that the granules are hard and/or non-flakable or breakable granules, in at least one version of the invention it is intended that they have a compressive strength of the values indicated above.

The tests for the determination of the compressive strength were carried out, for example, with a suitable machine consisting of a hollow cylinder containing the material under analysis, closed at the bottom by a metal base, and a piston for applying the load. The lower base in turn rests on a metal plate while the load is transmitted to the piston via a ball joint.

Experimental tests were carried out by the applicant on the granules object of the present invention and on prior art granules (described as flaky in the present treatment and/or, according to at least one example, including the granules known with the name of ParallaxTM granules) with this machine. Each test was repeated three times with three different specimens.

8 $cm^3$ of granules were gently poured into the cylinder. This volume of granules corresponds to about 8.55 grams for the prior art granules and about 10.22 grams for the granules of the present invention. With these granules, the hollow cylinder is filled to a height of 25 mm (L0).

Following the insertion of the granules into the cylinder, we proceeded with a slight vibration of the system for a few seconds, so that the granules in proximity to the loading piston were homogeneously arranged on a horizontal plane. Once the cylinder was placed in the test machine, an increasing compression load was started using a test speed of 1 mm/min. During the load application step, load (N) and load piston stroke ($\Delta$L) with a sampling rate (data acquisition frequency) of 0.05 Hz (20 data/second) have been continuously detected. The test was completed near the compressibility limit of the tested granules. This limit, since it cannot be quantified in absolute terms as the material, due to high compression values, tends to increasingly higher load values, was identified in a conventional way when the test graph began to assume a trend with a vertical asymptote. For each test, the percentage crushing was also evaluated, determined as:

$$n = \Delta L / L0 * 100$$

Where is it:

n = percentage crushing [%];
L0 = initial length, equal to 25 mm [mm];
$\Delta$L = displacement of the crosspiece [mm].

Observing the resulting graphs, for both the tested granules we observe a first section, in which the material reacts in a linear manner, and a second section with a progressively exponential trend due to the compaction of the various granules due to the compaction and approaching the limit of compressibility of the same. Given the trend observed, in order to characterize the behaviour of the granules, the values of Force (N) relating to two different percentages of compression (respectively equal to 10% and 20%) are reported in Table 2 and Table 3, for the two types of tested granules. In this way we remained in the part of the graph with a linear trend, where the two types of product can be compared before entering the zone with a non-linear trend and close to the compressibility limit of the product.

Table 2: Load corresponding to 10% and 20% compression of the granules of prior art

| Sample | Load corresponding to 10% of compression (N) | | Load corresponding to 20% of compression (N) | |
|---|---|---|---|---|
| 1 | 35 | Average Value | 90 | Average Value |
| 2 | 22 | | 69 | |
| 3 | 28 | **28** | 69 | **76** |

Table 3: Load corresponding to 10% and 20% compression of the granules of the present invention

| Sample | Load corresponding to 10% of compression (N) | | Load corresponding to 20% of compression (N) | |
|---|---|---|---|---|
| 1 | 1631 | Average Value | 4394 | Average Value |
| 2 | 1173 | | 3632 | |
| 3 | 1224 | **1343** | 3768 | **3931** |

The obtained results show a very different compressive strength for the two types of granules tested, since the average load corresponding to 10% of compression is equal to 28 N for the granules of prior art compared to 1343 N for the granules of the present invention, while the average compression load corresponding to 20% is equal to 76 N for the granules of the prior art compared to 3931 N for the granules of the present invention. According to at least one version, the granules of the present invention comprise barium sulphate and adhesive substance.

The second radiopaque agent comprises granules having a predefined a priori size. This is also because they do not break during transport, handling and use.

It has thus been seen how the present invention can adapt to specific surgical needs, providing a composition of a radiopaque agent which allows a better fluoroscopic and/or radiographic visibility to a filling material that contains it even on difficult patients, such as obese patients.

When the composition according to the present invention is added to the filling material, a homogeneous distribution of the first and second radiopaque agents is determined throughout the volume of the filling material. In this way, when implanted in the human body, the filling material will be visible in its entire volume. This is very important also in the case implant by injection, as even small quantities of the filler material will be clearly visible, as well as large quantities of it. This aspect is also very important in vertebroplasty, as the filling material cannot flow or be positioned outside the specific implant site, in order to avoid damage to the patient.

In a preferred embodiment, the filling material is a polymethylmethacrylate-based bone cement while the composition comprises barium sulphate in both powder and granule form.

Therefore, the present invention can also refer to a method for obtaining a filling material, comprising providing a radiopaque system according to the present invention and adding and mixing said radiopaque system to the filling material or to a powder phase of the filling material and/or to a liquid phase of the filler material, optionally add at least one pharmaceutical or medical substance to the filler material or a powder phase of the filler material and/or a liquid phase of the filler material, polymerize the filler material and/or react the powder phase and the liquid phase of the filler material for obtaining a filler material in the solidifiable fluid state. In such away, the mixing step determines a homogeneous distribution of the radiopaque system in the overall volume of obtainable filling material.

## Claims

1. Radiopaque system, adapted in use to be added to a filling material to be implanted in the human body to make this filling material visible when subjected to fluoroscopic and/or radiographic techniques, comprising at least a first radiopaque agent in the form of powder and at least a second radiopaque agent in the form of granules, in which said first radiopaque agent comprises at least one first radiopaque substance and in which said second radiopaque agent comprises at least one second radiopaque substance in the form of particles, in which said granules have a larger size than said powder, in wherein said at least one first radiopaque substance is equal to or different from said at least one second radiopaque substance, wherein said granules of said second radiopaque agent have at least a size or diameter between 600 microns and 850 microns and comprise said particles of said at least one second radiopaque substance and an adhesive substance, capable of compacting and holding together and/or gluing said particles of said at least one second radiopaque substance, wherein also said at least one first radiopaque agent comprises said adhesive substance, able to compact and hold together and/or glue particles of said at least one first radiopaque substance, **characterized by the fact that** said adhesive substance is homogeneously distributed within said granules, **in that** said second radiopaque agent in the form of granules comprises a percentage of between 1% and 30% by weight of said adhesive substance on the total weight of said second radiopaque agent, **and in that** said granules of said at least one second radiopaque agent are hard and non-flaky and have an average compressive strength of not less than 1000 N corresponding to 10% of compression or an average compressive strength of not less than 3000 N corresponding to 20% of compression, wherein the average compressive strength is obtained by means of a machine consisting of a hollow cylinder containing a volume of said granules, closed on the bottom by a metal base, and by a piston for applying an increasing compressive load force on said granules, evaluating the values of the load force, of the piston stroke and of the percentage of compression of said volume of said granules, and wherein said granules have a density between 1.15 and 1.35 g/cm$^3$, wherein said density is made by hydrostatic weighing of said granules of said at least a second radiopaque agent.

2. Radiopaque system according to claim 1, wherein said at least one second radiopaque agent and said first radiopaque agent are present in said radiopaque system in a ratio of approximately 3:1.

3. Radiopaque system according to claim 1, wherein said granules have a density equal to 1.25 g/cm$^3$, or equal to 1.27-1.28 g/cm$^3$, or equal to 1.277 g/cm$^3$, in which said density is made by hydrostatic weighing of said granules of said at least a second radiopaque agent, and/or wherein said powder of said at least one first radiopaque agent has a size or diameter comprised between 100 and 250 microns or lower than 100 microns and/or lower than 250 microns or between 0.1 microns and 10 micron and/or between 0.1 micron and 1 micron, said powder of said at least one first radiopaque agent comprising particles of said at least one first radiopaque substance.

4. Radiopaque system according to any one of previous claims, wherein said adhesive substance comprises a solid or a liquid, such as cellulose, polyvinyl alcohol, polyvinylpyrrolidone, carboxymethylcellulose, at least one sugar, polyvinyl acetate and analogues, synthetic and natural gums and resins, at least one resin acrylic, a methacrylic resin, a styrene resin or a mixture of at least two of the same, wherein said at least one resin is in the form of a polymer and/or co-polymer and/or a mixture of polymers and/or co-polymers and/or wherein said second radiopaque agent in the form of granules comprises 10% by weight of said adhesive substance on the total weight of said second radiopaque agent.

5. Radiopaque system according to any one of the preceding claims, wherein said powder of said at least one first radiopaque agent and/or said granules of said at least one second radiopaque agent have a substantially regular and/or irregular shape and/or a substantially spherical shape and/or similar to a parallelepiped, with rounded corners, and/or elongated or oblong, and/or oval or ovoid, and/or cylindrical with rounded bases, and/or similar to a "baguette", and/or needle-like.

6. Radiopaque system according to any one of the preceding claims, wherein said at least one first radiopaque substance and/or said at least one second radiopaque substance is barium sulphate, zirconium oxide, bismuth salt, tungsten powder, tantalum, a combination of the same.

7. Radiopaque system according to any one of the preceding claims, wherein both said at least one first radiopaque agent and said at least one second radiopaque agent is in dry and/or solid form and/or wherein said radiopaque system consists of said first radiopaque agent and said second radiopaque agent.

8. Filling material, suitable for implantation in the human body in vertebroplasty and/or in orthopaedics, comprising a plastic polymer and/or an acrylic resin, and/or a polymer based on PMMA and/or styrene and/or acrylic copolymers, and/or a ceramic bone cement, for example based on tricalcium phosphate (TCP) and/or hydroxyapatite, or a mixture thereof, **characterized by the fact of** comprising a radiopaque system according to any one of claims 1 to 7 adapted to make said filler material visible in use when subjected to fluoroscopic and/or radiographic techniques, wherein said system, comprising said first radiopaque agent in powder form and said second radiopaque agent in the form of granules, is uniformly distributed in said filling material.

9. Filling material according to claim 8, wherein said material is injectable and hardenable and/or polymerizable.

10. Filling material according to claim 8 or 9, wherein said radiopaque system is substantially present for 10% or 30% and/or is comprised between 5% and 50% or between 10% and 40% or between 20% and 30% by weight of the total weight of said filling material.

11. Filling material according to any one of claims 8 to 10, wherein said at least one first radiopaque agent constitutes 5-10% by weight or 7% by weight of the total weight of said filling material and/or wherein said at least a second radiopaque agent constitutes 10-30% by weight or 22% by weight of the total weight of said filling material.

12. Filling material according to any one of claims 8 to 11, comprising at least one pharmaceutical or medical substance, such as for example an antibiotic, such as for example gentamicin sulphate or vancomycin, or mixtures thereof, or a chemotherapeutic agent or an agent stimulating bone or tissue growth.

13. A kit comprising a radiopaque system according to any one of claims 1 to 7, wherein said radiopaque system comprises a package of at least one first radiopaque agent in the form of a powder and a package of at least a second radiopaque agent in the form of granules or a package comprising at least a first radiopaque agent in the form of a powder and at least a second radiopaque agent in the form of granules.

14. Kit according to claim 13, comprising a filling material suitable to be implanted in the human body in vertebroplasty and/or in orthopaedics, comprising a plastic polymer and/or an acrylic resin, and/or a polymer based on PMMA and/or styrene and/or acrylic copolymers, and/or a ceramic bone cement, for example based on tricalcium phosphate (TCP) and/or hydroxyapatite, or a mixture thereof.

15. Kit according to claim 14, wherein said filling material comprises a package for a powder phase of said filling material and an optional package comprising a liquid phase of said filling material and/or a package or vial containing at least one pharmaceutical substance or medical.

16. Method for obtaining a radiopaque system according to any one of claims 1 to 7, comprising the following steps:

provide at least a second radiopaque substance in the form of a powder,
providing a liquid solution comprising a solvent and an adhesive substance in solid form,
wherein said liquid solution contains 70-99% w/w of said liquid solvent and 30-1% w/w of said adhesive substance in solid form,
join and mix said at least one second radiopaque substance and said liquid solution, so as to obtain a wet aggregate mass, in the form of lumps having dimensions ranging for example between 3 mm and about 50 mm in diameter,

evaporate, for example in vacuum, said solvent so as to dry and consolidate said lumps thanks to said adhesive substance and obtain solidified lumps,

crushing said solidified lumps,

sieving the solidified and crushed lumps by means of a pair of sieves having dimensions of 600 microns and 850 microns so as to obtain granules of said at least one second radiopaque agent, in which said granules have a larger size than said powder, have at least a size or diameter between 600 microns and 850 microns and comprise said adhesive substance homogeneously distributed within said granules, wherein said second radiopaque agent in the form of granules comprises a percentage of between 1% and 30% by weight of said adhesive substance on the total weight of said second radiopaque agent, providing said at least one first radiopaque agent in powder form, combining said at least one first radiopaque substance with a liquid solution comprising a solvent and an adhesive substance in solid form, evaporate said solvent present in said liquid solution, solidify and/or consolidate said particles of said at least one first radiopaque substance and obtain said powder of said at least one first radiopaque agent, and adding it to said granules of said at least one second radiopaque agent.

17. Method according to claim 16, wherein said solidified and crushed lumps have at least one dimension and/or diameter whose measurement is according to a Gaussian or Gaussian-like distribution, and/or wherein said solidified and crushed lumps are present for approximately 20-22% with a particle size of less than 600 microns, approximately 35-36% with a particle size between 600 microns and 850 microns and approximately 40-42% with a particle size greater than 850 microns, and/or in which said at least one second radiopaque substance is supplied in a percentage equal to 70-97% w/w (weight/weight) of the total while said liquid solution comprising said adhesive substance is supplied in a percentage equal to 3-30 % w/w of the total, considering the total given by the sum of said at least one second radiopaque substance and of said liquid solution.

18. Method according to claim 16 or 17, wherein said liquid solution is prepared by combining at least one solvent, such as for example a polar or non-polar liquid and/or solvent or a mixture between the two types, or water, ethanol, methanol, acetone, ether, hexane, at least one acrylic, methacrylic or styrene monomer and said adhesive substance in solid form.

19. Method according to any one of claims 16 to 18, wherein said step of providing said at least one first radiopaque agent comprises providing at least one first radiopaque substance in the form of a powder, sieving said at least one first radiopaque substance so as to select particles of said powder of said at least one radiopaque substance.

20. Method for obtaining a filling material according to any one of claims 8 to 12, comprising providing a radiopaque system according to any one of claims 1 to 7 and adding and mixing said radiopaque system to said filling material or to a powder phase of said filling material and/or a liquid phase of said filling material, optionally adding at least one pharmaceutical or medical substance to said filling material or to a powder phase of said filling material and/or to a liquid phase of said filling material, polymerizing said filling material and/or causing said powder phase and said liquid phase of said filling material to react to obtain a filling material in the solidifiable fluid state.

21. Use of the radiopaque system according to one or more of claims 1 to 7, as an additive system for making radiopaque a filling material suitable for implantation in the human body in vertebroplasty and/or in orthopaedics.

22. Filling material according to any one of claims 8 to 12, for use in fluoroscopy or radiography in obese patients, in which it is very difficult to distinguish the filling material that is implanted.

**Patentansprüche**

1. Röntgenopakes System, das dazu ausgelegt ist, im Gebrauch einem in den menschlichen Körper zu implantierenden Füllmaterial zugesetzt zu werden, um dieses Füllmaterial sichtbar zu machen, wenn es fluoroskopischen und/oder radiographischen Techniken unterzogen wird, umfassend mindestens ein erstes röntgenopakes Mittel in Form von Pulver und mindestens ein zweites röntgenopakes Mittel in Form von Granulaten, in dem das besagte erste röntgenopake Mittel mindestens eine erste röntgenopake Substanz umfasst und in dem das besagte zweite röntgenopake Mittel mindestens eine zweite röntgenopake Substanz in Form von Partikeln umfasst, in dem die besagten Granulate eine größere Größe als das besagte Pulver haben, worin die besagte mindestens eine erste röntgenopake Substanz gleich oder verschieden von der besagten mindestens einen zweiten röntgenopaken Substanz ist, worin die besagten Granulate des besagten zweiten röntgenopaken Mittels mindestens eine Größe oder einen Durchmesser zwischen 600 Mikrometern und 850 Mikrometern haben und die besagten Partikel der besagten mindestens einen zweiten röntgenopaken Substanz und eine Klebesubstanz umfassen, die in der Lage ist, die besagten Partikel der besagten mindestens einen zweiten röntgenopaken Substanz zu verdichten und zusammenzuhalten und/oder zu verkleben, worin auch das besagte mindestens eine erste röntgenopake Mittel die besagte Klebesubstanz umfasst, der in der Lage ist, die Partikel der besagten mindestens einen ersten röntgenopaken Substanz zu verdichten und zusammenzuhalten und/oder zu verkleben, **dadurch gekennzeichnet, dass** die besagte Klebesubstanz homogen in den besagten Partikeln verteilt ist, dadurch, dass das besagte zweite röntgenopake Mittel in Form von Granulaten einen Prozentsatz zwischen 1 und 30 Gew.-% der besagten Klebesubstanz auf das Gesamtgewicht des besagten zweiten röntgenopaken Mittels umfasst, und dadurch, dass die besagten Granulate des besagten mindestens einen zweiten röntgenopaken Mittels hart und nicht flockig sind und eine mittlere Druckfestigkeit von nicht weniger als 1000 N entsprechend 10 % Kompression oder eine mittlere Druckfestigkeit von nicht weniger 3000 N entsprechend 20 % Kompression aufweisen, worin die mittlere Druckfestigkeit durch eine Maschine, bestehend aus einem ein Volumen der besagten Granulate enthaltenden Hohlzylinder, der am Boden durch eine Metallbasis verschlossen ist, und durch einen Kolben zum Aufbringen einer zunehmenden Druckbelastungskraft auf die besagten Granulat erhalten wird, wobei die Werte der Belastungskraft, des Kolbenhubs und des Prozentsatzes der Kompression des besagten Volumens der besagten Granulate bewertet werden, und worin die besagten Granulate eine Dichte zwischen 1,15 und 1,35 g/cm$^3$ aufweisen, worin die besagte Dichte durch hydrostatisches Wiegen der besagten Granulate des besagten mindestens einen zweiten röntgenopaken Mittels bestimmt wird.

2. Röntgenopakes System nach Anspruch 1, worin das besagte mindestens eine zweite röntgenopake Mittel und das besagte erste röntgenopake Mittel in dem besagten röntgenopaken System in einem Verhältnis von etwa 3:1 vorhanden sind.

3. Röntgenopakes System nach Anspruch 1, worin die besagten Granulate eine Dichte von 1,25 g/cm$^3$ oder von 1,27-1,28 g/cm$^3$ oder von 1,277 g/cm$^3$ haben, in dem die besagte Dichte durch hydrostatisches Wiegen der besagten Granulate des besagten mindestens einen zweiten röntgenopaken Mittels hergestellt wird, und/oder worin das besagte Pulver des besagten mindestens einen ersten röntgenopaken Mittels eine Größe oder einen Durchmesser zwischen 100 und 250 Mikrometer oder weniger als 100 Mikrometer und/oder weniger als 250 Mikrometer oder zwischen 0,1 Mikrometer und 10 Mikrometer und/oder zwischen 0,1 Mikrometer und 1 Mikrometer hat, wobei das besagte Pulver des besagten mindestens einen ersten röntgenopaken Mittels Partikel der besagten mindestens einen ersten röntgenopaken Substanz umfasst.

4. Röntgenopakes System nach irgendeinem der vorangegangenen Ansprüche, worin die besagte Klebesubstanz einen Feststoff oder eine Flüssigkeit umfasst, wie Cellulose, Polyvinylalkohol, Polyvinylpyrrolidon, Carboxymethylcellulose, mindestens einen Zucker, Polyvinylacetat und Analoga, synthetische und natürliche Gummis und Harze, mindestens ein Acrylharz, ein Methacrylharz, ein Styrolharz oder eine Mischung von mindestens zwei derselben, worin das besagte mindestens eine Harz in Form eines Polymers und/oder Copolymers und/oder einer Mischung von Polymeren und/oder Copolymeren vorliegt und/oder worin das besagte zweite röntgenopake Mittel in Form von Granulaten 10 Gew.-% der besagten Klebesubstanz auf das Gesamtgewicht des besagten zweiten röntgenopaken Mittels umfasst.

5. Röntgenopakes System nach irgendeinem der vorangegangenen Ansprüche, worin das besagte Pulver des besagten mindestens einen ersten röntgenopaken Mittels und/oder der besagten Granulate des besagten mindestens einen zweiten röntgenopaken Mittels eine im Wesentlichen regelmäßige und/oder unregelmäßige Form und/oder eine im Wesentlichen kugelförmige und/oder einem Parallelepiped ähnliche, mit abgerundeten Ecken, und/oder langgestreckte oder längliche und/oder ovale oder eiförmige und/oder zylindrische mit abgerundeten Basen und/oder einem "Baguette" ähnliche und/oder nadelartige Form haben.

6. Röntgenopakes System nach irgendeinem der vorangegangenen Ansprüche, worin die besagte mindestens eine erste röntgenopake Substanz und/oder die besagte mindestens eine zweite röntgenopake Substanz Bariumsulfat, Zirkoniumoxid, Bismutsalz, Wolframpulver, Tantal, eine Kombination davon ist.

7. Röntgenopakes System nach irgendeinem der vorangegangenen Ansprüche, worin sowohl das besagte mindestens eine erste röntgenopake Mittel als auch das besagte mindestens eine zweite röntgenopake Mittel in trockener und/oder fester Form vorliegt und/oder worin das besagte röntgenopake System aus dem besagten ersten röntgenopaken Mittel und dem besagten zweiten röntgenopaken Mittel besteht.

8. Füllmaterial, geeignet zur Implantation in den menschlichen Körper bei der Vertebroplastie und/oder in der Orthopädie, umfassend ein Kunststoffpolymer und/oder ein Acrylharz, und/oder ein Polymer auf der Basis von PMMA und/oder Styrol und/oder Acrylcopolymeren, und/oder einen keramischen Knochenzement, zum Beispiel auf der Basis von Tricalciumphosphat (TCP) und/oder Hydroxyapatit, oder eine Mischung davon, **dadurch gekennzeichnet, dass** es ein röntgenopakes System nach einem der Ansprüche 1 bis 7 umfasst, das dazu ausgelegt ist, das besagte Füllmaterial im Gebrauch sichtbar zu machen, wenn es fluoroskopischen und/oder radiographischen Techniken unterzogen wird, worin das besagte System, umfassend das besagte erste röntgenopake Mittel in Pulverform und das besagte zweite röntgenopake Mittel in Form von Granulaten, gleichmäßig in dem besagten Füllmaterial verteilt ist.

9. Füllmaterial nach Anspruch 8, worin das besagte Material injizierbar und härtbar und/oder polymerisierbar ist.

10. Füllmaterial nach Anspruch 8 oder 9, worin das besagte röntgenopake System im Wesentlichen zu 10 % oder 30 % vorhanden ist und/oder zwischen 5 und 50 oder zwischen 10 und 40 oder zwischen 20 und 30 Gew.-% des Gesamtgewichts des besagten Füllmaterials liegt.

11. Füllmaterial nach irgendeinem der Ansprüche 8 bis 10, worin das besagte mindestens eine erste röntgenopake Mittel 5-10 Gew.-% oder 7 Gew.-% des Gesamtgewichts des besagten Füllmaterials ausmacht und/oder worin das besagte mindestens eine zweite röntgenopake Mittel 10-30 Gew.-% oder 22 Gew.-% des Gesamtgewichts des besagten Füllmaterials ausmacht.

12. Füllmaterial nach irgendeinem der Ansprüche 8 bis 11, umfassend mindestens eine pharmazeutische oder medizinische Substanz, wie zum Beispiel ein Antibiotikum, wie zum Beispiel Gentamicinsulfat oder Vancomycin, oder Mischungen davon, oder ein chemotherapeutisches Mittel oder ein das Knochen- oder Gewebewachstum stimulierendes Mittel.

13. Kit, umfassend ein röntgenopakes System nach irgendeinem der Ansprüche 1 bis 7, worin das besagte röntgenopake System eine Packung mit mindestens einem ersten röntgenopaken Mittel in Form eines Pulvers und eine Packung mit mindestens einem zweiten röntgenopaken Mittel in Form von Granulaten oder eine Packung mit mindestens einem ersten röntgenopaken Mittel in Form eines Pulvers und mindestens einem zweiten röntgenopaken Mittel in Form von Granulaten umfasst.

14. Kit nach Anspruch 13, umfassend ein Füllmaterial, das geeignet ist, bei der Vertebroplastie und/oder in der Orthopädie in den menschlichen Körper implantiert zu werden, umfassend ein Kunststoffpolymer und/oder ein Acrylharz, und/oder ein Polymer auf der Basis von PMMA und/oder Styrol und/oder Acrylcopolymeren, und/oder einen keramischen Knochenzement, zum Beispiel auf der Basis von Tricalciumphosphat (TCP) und/oder Hydroxyapatit, oder eine Mischung davon.

15. Kit nach Anspruch 14, worin das besagte Füllmaterial eine Packung für eine Pulverphase des besagten Füllmaterials und eine optionale Packung, umfassend eine Flüssigphase des besagten Füllmaterials, und/oder eine Packung oder eine Phiole, enthaltend mindestens eine pharmazeutische oder medizinische Substanz, umfasst.

16. Verfahren zur Erhaltung eines röntgenopaken Systems nach irgendeinem der Ansprüche 1 bis 7, umfassend die folgenden Schritte:

des Bereitstellens mindestens einer zweiten röntgenopaken Substanz in Form eines Pulvers,
des Bereitstellens einer flüssigen Lösung, umfassend ein Lösungsmittel und eine Klebesubstanz in fester Form, worin die besagte flüssige Lösung 70-99 w/w% des besagten flüssigen Lösungsmittels und 30-1 w/w% der besagten Klebesubstanz in fester Form enthält,
des Verbindens und des Vermischens der besagten mindestens einen zweiten röntgenopaken Substanz und der besagten flüssigen Lösung, um eine feuchte Aggregatmasse in Form von Klumpen zu erhalten, deren Abmessungen zum Beispiel zwischen 3 mm und etwa 50 mm im Durchmesser liegen,
des Verdampfens, zum Beispiel im Vakuum, des besagten Lösungsmittels, um die besagten Klumpen dank der besagten Klebesubstanz zu trocknen und zu verdichten und verfestigte Klumpen zu erhalten,
des Zerkleinerns der besagten verfestigten Klumpen,
des Siebens der verfestigten und zerkleinerten Klumpen mittels eines Siebpaares mit Abmessungen von 600 Mikrometern und 850 Mikrometern, um Granulate des besagten mindestens einen zweiten röntgenopaken Mittels zu erhalten, in dem die besagten Granulate eine größere Größe als das besagte Pulver haben, mindestens eine Größe oder einen Durchmesser zwischen 600 Mikrometer und 850 Mikrometer haben und die besagte Klebesubstanz homogen in den besagten Granulaten verteilt umfassen, worin das besagte zweite röntgenopake Mittel in Form von Granulaten einen Prozentsatz zwischen 1 und 30 Gew.-% der besagten Klebesubstanz auf das Gesamtgewicht des besagten zweiten röntgenopaken Mittels umfasst, des Bereitstellens des besagten mindestens einen ersten röntgenopaken Mittels in Pulverform, des Kombinierens des besagten mindestens einen ersten röntgenopaken Mittels mit einer flüssigen Lösung, umfassend ein Lösungsmittel und eine Klebesubstanz in fester Form, des Verdampfens des besagten Lösungsmittels, das in der besagten flüssigen Lösung vorhanden ist, des Verfestigens und/oder des Verdichtens der besagten Partikel des besagten mindestens einen ersten röntgenopaken Mittels und des Erhaltens des besagten Pulvers des besagten mindestens einen ersten röntgenopaken Mittels, und des Zusetzens desselben zu den besagten Granulaten des besagten mindestens einen zweiten röntgenopaken Mittels.

17. Verfahren nach Anspruch 16, worin die besagten verfestigten und zerkleinerten Klumpen mindestens eine Abmessung und/oder einen Durchmesser aufweisen, deren bzw. dessen Messung einer Gauß'schen oder Gauß-artigen Verteilung entspricht, und/oder worin die besagten verfestigten und zerkleinerten Klumpen zu etwa 20-22% mit einer Partikelgröße von weniger als 600 Mikrometern, zu etwa 35-36% mit einer Partikelgröße zwischen 600 Mikrometern und 850 Mikrometern und zu etwa 40-42% mit einer Partikelgröße von mehr als 850 Mikrometern vorhanden sind, und/oder in dem die besagte mindestens eine zweite röntgenopake Substanz in einem Prozentsatz von 70-97 w/w% (Gewicht/Gewicht) der Gesamtmenge zugeführt wird, während die besagte flüssige Lösung, umfassend die besagte Klebesubstanz, in einem Prozentsatz von 3-30 w/w% der Gesamtmenge zugeführt wird, wobei die Gesamtmenge durch die Summe der besagten mindestens einen zweiten röntgenopaken Substanz und der besagten flüssigen Lösung gegeben ist.

18. Verfahren nach Anspruch 16 oder 17, worin die besagte flüssige Lösung hergestellt wird, indem mindestens ein Lösungsmittel, wie zum Beispiel eine polare oder unpolare Flüssigkeit und/oder ein Lösungsmittel oder eine Mischung zwischen den beiden Arten, oder Wasser, Ethanol, Methanol, Aceton, Ether, Hexan, mindestens ein Acryl-, Methacryl- oder Styrolmonomer und die besagte Klebesubstanz in fester Form kombiniert werden.

19. Verfahren nach irgendeinem der Ansprüche 16 bis 18, worin der besagte Schritt des Bereitstellens des besagten mindestens einen ersten röntgenopaken Mittels das Bereitstellen mindestens einer ersten röntgenopaken Substanz in Form eines Pulvers, das Sieben der besagten mindestens einen ersten röntgenopaken Substanz, um Partikel des besagten Pulvers der besagten mindestens einen röntgenopaken Substanz auszuwählen, umfasst.

20. Verfahren zur Erhaltung eines Füllmaterials nach irgendeinem der Ansprüche 8 bis 12, umfassend das Bereitstellen eines röntgenopaken Systems nach irgendeinem der Ansprüche 1 bis 7 und das Zusetzen und Mischen des besagten röntgenopaken Systems zu dem besagten Füllmaterial oder zu einer Pulverphase des besagten Füllmaterials und/oder einer Flüssigphase des besagten Füllmaterials, wahlweise das Zusetzen mindestens einer pharmazeutischen oder medizinischen Substanz zu dem besagten Füllmaterial oder zu einer Pulverphase des besagten Füllmaterials und/oder zu einer Flüssigphase des besagten Füllmaterials, das Polymerisieren des besagten Füllmaterials und/oder das Reagierenlassen der besagten Pulverphase und der besagten Flüssigphase des besagten Füllmaterials, um ein Füllmaterial im verfestigbaren flüssigen Zustand zu erhalten.

21. Verwendung des röntgenopaken Systems nach einem oder mehreren der Ansprüche 1 bis 7 als ein additives System, um ein Füllmaterial, das zur Implantation in den menschlichen Körper bei der Vertebroplastie und/oder in der Orthopädie geeignet ist, röntgenopak zu machen.

22. Füllmaterial nach irgendeinem der Ansprüche 8 bis 12 zum Gebrauch in der Fluoroskopie oder Radiographie bei adipösen Patienten, bei denen es sehr schwierig ist, das Füllmaterial, das implantiert wird, zu unterscheiden.

**Revendications**

1. Système radio-opaque, adapté en cours d'utilisation à être ajouté à un matériau de remplissage destiné à être implanté dans le corps humain afin de rendre ce matériau de remplissage visible lorsqu'il est soumis à des techniques fluoroscopiques et/ou radiographiques, comprenant au moins un premier agent radio-opaque sous forme de poudre et au moins un deuxième agent radio-opaque sous forme de granules, dans lequel ledit premier agent radio-opaque comprend au moins une première substance radio-opaque et dans lequel ledit deuxième agent radio-opaque comprend au moins une deuxième substance radio-opaque sous forme de particules, dans lequel lesdits granules ont une taille plus grande que ladite poudre, dans lequel ladite au moins une première substance radio-opaque est égale à ou différente de ladite au moins une deuxième substance radio-opaque, dans lequel lesdits granules dudit deuxième agent radio-opaque ont au moins une taille ou un diamètre compris entre 600 microns et 850 microns et comprennent lesdites particules de ladite au moins une deuxième substance radio-opaque et une substance adhésive, capables de compacter et de maintenir ensemble et/ou de coller lesdites particules de ladite au moins une deuxième substance radio-opaque, dans lequel également ledit au moins un premier agent radio-opaque comprend ladite substance adhésive, capable de compacter et de maintenir ensemble et/ou de coller des particules de ladite au moins une première substance radio-opaque, **caractérisée en ce que** ladite substance adhésive est répartie de manière homogène à l'intérieur desdits granules, **en ce que** ledit deuxième agent radio-opaque sous forme de granules comprend un pourcentage compris entre 1 % et 30 % en poids de ladite substance adhésive sur le poids total dudit deuxième agent radio-opaque, et **en ce que** lesdits granules dudit au moins un deuxième agent radio-opaque sont durs et non feuilletés et ont une résistance à la compression moyenne d'au moins 1000 N correspondant à 10 % de compression ou une résistance à la compression moyenne d'au moins 3000 N correspondant à 20 % de compression, dans lequel la résistance à la compression moyenne est obtenue au moyen d'une machine constituée d'un cylindre creux contenant un volume desdits granules, fermé sur le fond par une base métallique, et par un piston pour appliquer une force de charge de compression croissante sur lesdits granules, en évaluant les valeurs de la force de charge, de la course du piston et du pourcentage de compression dudit volume desdits granules, et dans lequel lesdits granules ont une densité comprise entre 1,15 et 1,35 g/cm$^3$, dans lequel ladite densité est obtenue par pesage hydrostatique desdits granules dudit au moins un deuxième agent radio-opaque.

2. Système radio-opaque selon la revendication 1, dans lequel ledit au moins un deuxième agent radio-opaque et ledit premier agent radio-opaque sont présents dans ledit système radio-opaque dans un rapport d'environ 3:1.

3. Système radio-opaque selon la revendication 1, dans lequel lesdits granules ont une densité égale à 1,25 g/cm$^3$, ou égale à 1,27-1,28 g/cm$^3$, ou égale à 1,277 g/cm$^3$, dans lequel ladite densité est obtenue par pesée hydrostatique desdits granules dudit au moins un deuxième agent radio-opaque, et/ou dans lequel ladite poudre dudit au moins un premier agent radio-opaque a une taille ou un diamètre compris entre 100 et 250 microns ou inférieur à 100 microns et/ou inférieur à 250 microns ou entre 0,1 micron et 10 microns et/ou entre 0,1 micron et 1 micron, ladite poudre dudit au moins un premier agent radio-opaque comprenant des particules de ladite au moins une première substance radio-opaque.

4. Système radio-opaque selon l'une quelconque des revendications précédentes, dans lequel ladite substance adhésive comprend un solide ou un liquide, tel que la cellulose, l'alcool polyvinylique, la polyvinylpyrrolidone, la carboxyméthylcellulose, au moins un sucre, l'acétate de polyvinyle et ses analogues, des gommes et des résines synthétiques et naturelles, au moins une résine acrylique, une résine méthacrylique, une résine de styrène ou un mélange d'au moins deux de ces substances, dans lequel ladite au moins une résine se présente sous la forme d'un polymère et/ou copolymère et/ou d'un mélange de polymères et/ou de copolymères et/ou dans lequel ledit deuxième agent radio-opaque sous forme de granules comprend 10 % en poids de ladite substance adhésive sur le poids total dudit deuxième agent radio-opaque.

5. Système radio-opaque selon l'une quelconque des revendications précédentes, dans lequel ladite poudre dudit au moins un premier agent radio-opaque et/ou lesdits granules dudit au moins un deuxième agent radio-opaque ont une forme sensiblement régulière et/ou irrégulière et/ou une forme sensiblement sphérique et/ou similaire à un parallélépipède, avec des coins arrondis, et/ou allongé ou oblong, et/ou ovale ou ovoïde, et/ou cylindrique à bases arrondies, et/ou semblable à une « baguette », et/ou en forme d'aiguille.

6. Système radio-opaque selon l'une quelconque des revendications précédentes, dans lequel ladite au moins une première substance radio-opaque et/ou ladite au moins une deuxième substance radio-opaque est du sulfate de baryum, de l'oxyde de zirconium, du sel de bismuth, de la poudre de tungstène, du tantale, une combinaison de ces substances.

7. Système radio-opaque selon l'une quelconque des revendications précédentes, dans lequel ledit au moins un premier agent radio-opaque et ledit au moins un deuxième agent radio-opaque sont sous forme sèche et/ou solide et/ou dans lequel ledit système radio-opaque se compose dudit premier agent radio-opaque et dudit deuxième agent radio-opaque.

8. Matériau de remplissage, apte à être implanté dans le corps humain en vertébroplastie et/ou en orthopédie, comprenant un polymère plastique et/ou une résine acrylique, et/ou un polymère à base de PMMA et/ou de styrène et/ou de copolymères acryliques, et/ou un ciment osseux céramique, par exemple à base de phosphate tricalcique (TCP) et/ou d'hydroxyapatite, ou un mélange de ceux-ci, **caractérisé par** le fait de comprendre un système radio-opaque selon l'une quelconque des revendications de 1 à 7 adapté pour rendre ledit matériau de remplissage visible en cours d'utilisation lorsqu'il est soumis à des techniques fluoroscopiques et/ou radiographiques, dans lequel ledit système, comprenant ledit premier agent radio-opaque sous forme de poudre et ledit deuxième agent radio-opaque sous forme de granules, est uniformément réparti dans ledit matériau de remplissage.

9. Matériau de remplissage selon la revendication 8, dans lequel ledit matériau est injectable et durcissable et/ou polymérisable.

10. Matériau de remplissage selon la revendication 8 ou 9, dans lequel ledit système radio-opaque est sensiblement présent pour 10 % ou 30 % et/ou est composé entre 5 % et 50 % ou entre 10 % et 40 % ou entre 20 % et 30 % en poids du poids total dudit matériau de remplissage.

11. Matériau de remplissage selon l'une quelconque des revendications 8 à 10, dans lequel ledit au moins un premier agent radio-opaque constitue 5 à 10 % en poids ou 7 % en poids du poids total dudit matériau de remplissage et/ou dans lequel ledit au moins un deuxième agent radio-opaque constitue 10 à 30 % en poids ou 22 % en poids du poids total dudit matériau de remplissage.

12. Matériau de remplissage selon l'une quelconque des revendications 8 à 11, comprenant au moins une substance pharmaceutique ou médicale, telle que par exemple un antibiotique, comme par exemple le sulfate de gentamicine ou la vancomycine, ou des mélanges de ceux-ci, ou un agent chimiothérapeutique ou un agent stimulant la croissance osseuse ou tissulaire.

13. Kit comprenant un système radio-opaque selon l'une quelconque des revendications 1 à 7, dans lequel ledit système radio-opaque comprend un emballage d'au moins un premier agent radio-opaque sous la forme d'une poudre et un emballage d'au moins un deuxième agent radio-opaque sous forme de granules ou un emballage comprenant au moins un premier agent radio-opaque sous la forme d'une poudre et au moins un deuxième agent radio-opaque sous forme de granules.

14. Kit selon la revendication 13, comprenant un matériau de remplissage apte à être implanté dans le corps humain en vertébroplastie et/ou en orthopédie, comprenant un polymère plastique et/ou une résine acrylique, et/ou un polymère à base de PMMA et/ou de styrène et/ou de copolymères acryliques, et/ou un ciment osseux céramique, par exemple à base de phosphate tricalcique (TCP) et/ou d'hydroxyapatite, ou un mélange de ceux-ci.

15. Kit selon la revendication 14, dans lequel ledit matériau de remplissage comprend un emballage pour une phase en poudre dudit matériau de remplissage et un emballage optionnel comprenant une phase liquide dudit matériau de remplissage et/ou un emballage ou un flacon contenant au moins une substance pharmaceutique ou médicale.

16. Procédé d'obtention d'un système radio-opaque selon l'une quelconque des revendications 1 à 7, comprenant les étapes suivantes :

fournir au moins une deuxième substance radio-opaque sous la forme d'une poudre,
fournir une solution liquide comprenant un solvant et une substance adhésive sous forme solide, dans laquelle ladite solution liquide contient 70-99 % p/p dudit solvant liquide et 30-1 % p/p de ladite substance adhésive sous forme solide,
joindre et mélanger ladite au moins une deuxième substance radio-opaque et ladite solution liquide, de manière à obtenir une masse agrégée humide, sous forme de grumeaux dont les dimensions varient par exemple entre 3 mm et environ 50 mm de diamètre,
évaporer, par exemple sous vide, ledit solvant de manière à sécher et consolider lesdits grumeaux grâce à ladite substance adhésive et obtenir des grumeaux solidifiés,
écraser lesdits morceaux solidifiés,
tamiser les morceaux solidifiés et broyés au moyen d'une paire de tamis de dimensions de 600 microns et 850 microns de manière à obtenir des granules dudit au moins un deuxième agent radio-opaque, dans lequel lesdits granules ont une taille plus grande que ladite poudre, ont au moins une taille ou un diamètre compris entre 600 microns et 850 microns et comprennent ladite substance adhésive répartie de manière homogène à l'intérieur desdits granules, dans lequel ledit deuxième agent radio-opaque sous forme de granules comprend un pourcentage compris entre 1 % et 30 % en poids de ladite substance adhésive sur le poids total dudit deuxième agent radio-opaque, fournir ledit au moins un premier agent radio-opaque sous forme de poudre, combiner ladite au moins une première substance radio-opaque avec une solution liquide comprenant un solvant et une substance adhésive sous forme solide, évaporer ledit solvant présent dans ladite solution liquide, solidifier et/ou consolider lesdites particules de ladite au moins une première substance radio-opaque et obtenir ladite poudre dudit au moins un premier agent radio-opaque, et l'ajouter auxdits granules dudit au moins un deuxième agent radio-opaque.

17. Procédé selon la revendication 16, dans lequel lesdits morceaux solidifiés et broyés ont au moins une dimension et/ou un diamètre dont la mesure est conforme à une distribution gaussienne ou de type gaussien, et/ou dans lequel lesdits morceaux solidifiés et broyés sont présents pendant environ 20 à 22 % avec une taille de particule inférieure à 600 microns, environ 35 à 36 % avec une taille de particule comprise entre 600 microns et 850 microns et environ 40 à 42 % avec une taille de particule supérieure à 850 microns, et/ou dans lequel ladite au moins une deuxième substance radio-opaque est fournie dans un pourcentage égal à 70-97 % p/p (poids/poids) du total, tandis que ladite solution liquide comprenant ladite substance adhésive est fournie dans un pourcentage égal à 3-30 % p/p du total, compte tenu du total donné par la somme de ladite au moins une deuxième substance radio-opaque et de ladite solution liquide.

18. Procédé selon la revendication 16 ou 17, dans lequel ladite solution liquide est préparée en combinant au moins un solvant, tel que par exemple un liquide et/ou un solvant polaire ou non polaire ou un mélange entre les deux types, ou de l'eau, de l'éthanol, du méthanol, de l'acétone, de l'éther, de l'hexane, au moins un monomère acrylique, méthacrylique ou styrène et ladite substance adhésive sous forme solide.

19. Procédé selon l'une quelconque des revendications 16 à 18, dans lequel ladite étape consistant à fournir ledit au moins un premier agent radio-opaque comprend l'administration d'au moins une première substance radio-opaque sous la forme d'une poudre, le tamisage de ladite au moins une première substance radio-opaque de manière à sélectionner des particules de ladite poudre de ladite au moins une substance radio-opaque.

20. Procédé d'obtention d'un matériau de remplissage selon l'une quelconque des revendications 8 à 12, comprenant la fourniture d'un système radio-opaque selon l'une quelconque des revendications 1 à 7 et l'ajout et le mélange dudit système radio-opaque audit matériau de remplissage ou à une phase poudreuse dudit matériau de remplissage et/ou à une phase liquide dudit matériau de remplissage, l'ajout facultatif d'au moins une substance pharmaceutique ou médicale audit matériau de remplissage ou à une phase en poudre dudit matériau de remplissage et/ou à une phase liquide dudit matériau de remplissage, en polymérisant ledit matériau de remplissage et/ou en provoquant la réaction de ladite phase en poudre et de ladite phase liquide dudit matériau de remplissage pour obtenir un matériau de remplissage à l'état fluide solidifiable.

21. Utilisation du système radio-opaque selon l'une ou plusieurs des revendications 1 à 7, en tant que système additif pour la fabrication d'un matériau de remplissage radio-opaque apte à être implanté dans le corps humain en vertébroplastie et/ou en orthopédie.

22. Matériau de remplissage selon l'une quelconque des revendications 8 à 12, pour une utilisation en fluoroscopie ou en radiographie chez les patients obèses, dans laquelle il est très difficile de distinguer le matériau de remplissage qui est implanté.

Fig. 1

Fig. 2

Fig. 3

(PRIOR ART)

Fig. 4

Fig. 5

Fig. 6

Fig. 7

Fig. 8

Fig. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004157952 A1
- US 2016279289 A